# EUROPEAN PATENT APPLICATION

(11) **EP 3 824 948 A1**
(43) Date of publication of application: **26.05.2021**
(21) Application number: 19209911.7
(22) Date of filing: 19.11.2019
(51) Int. Cl.: A61N 1/36, A61N 1/372, G16H 20/30, G16H 40/63

(54) **A PLANNING AND/OR CONTROL SYSTEM FOR A NEUROMODULATION SYSTEM**

(71) Applicant: ONWARD Medical B.V., 5656 AE Eindhoven (NL)
(72) Inventor: BROUNS, Robin, 5656 AE Eindhoven (NL); BAKKER, Jurriaan, 5656 AE Eindhoven (NL); CABAN, Miroslav, 1020 Renens (CH)
(74) Representative: DTS Patent- und Rechtsanwälte Schnekenbühl und Partner mbB

(57) **Abstract**

A planning and/or control system (10) for a system for providing neuromodulation, especially neurostimulation, at least comprising:
- a graphical presentation module (12) configured and arranged for providing graphical information about an electrode array (A) comprising multiple electrodes (E, V) and/or an implantation side for the electrode array comprising at least one target area (X),
- a selection module (14) configured and arranged for determining a stimulation zone (Z) and/or a stimulation direction (D) on the electrode array (A) comprising at least one electrode (E, V) and/or for individually selecting at least one electrode (E, V) and/or for selecting at least one target area (X),
- a calculation module (16) configured and arranged for determining a contribution of currents provided by electrodes (E;V) of the stimulation zone (Z) and/or stimulation direction (D) on the electrode array (A) and/or the at least one electrode (E, V) selected and/or to the at least one target area (X) selected.

## Description

The present Invention relates to control systems for tissue stimulating systems, in particular a planning and/or control system for a system for providing neuromodulation, especially neurostimulatlon for a patient.

The present invention further relates to a method for planning neuromodulation, especially neurostimulation for a patient.

The spinal cord is an integral part of the central nervous system (CNS). Spinal cord injury (SCI), but also other disorders (e.g. stroke, multiple sclerosis, autonomic failure, autonomic neuropathy or cancer of the neurological tissue which impair operation of descending sympathetic pathways that normally facilitate control of autonomic functions) result in motor deficits. For instance, SCI interrupts the communication between the spinal cord and supraspinal centres, depriving these sensorimotor circuits from the excitatory and modulatory drives necessary to produce movement. However, SCI also results in sensory deficits and In autonomic dysfunctions. In particular, SCI results in disconnection of some, most, or all descending sympathetic pathways that carry signals responsible for regulating arterial blood pressure, heart rate and/or gut and/or bladder function.

Spinal cord stimulation (SCS) is a well-established neuromodulatory therapy not only for restoring locomotion/motoric function after spinal cord injury or central nervous diseases, but also for treating inter alia pain and/or restoring autonomic function.

Neuromodulation, in particular neurostimulation, in particular SCS is typically applied to a subject by a neuromodulation system comprising at least one electrode array comprising at least one electrode. Neuromodulation systems may further comprise at least one of a controller, e.g. a microcontroller, a processor, e.g. a microprocessor, a pulse generator, In particular an Implantable pulse generator, a sensor, a communication module, a telemetry module.

The electrode array, e.g. comprised in a lead paddle, can be applied for percutaneous electrical stimulation, transcutaneous electrical nerve stimulation (TENS), epidural electrical stimulation (EES), subdural electrical stimulation (SES), functional electrical stimulation (FES) and/or all neurostimulation and/or muscle stimulation applications that use at least one electrode array and/or at least one electrode.

Lead paddles are for example known from US8108051B2, US 2013/0096662 A1, US 2012/0006793A1 and EP3013411A1.

A neuromodulation system, especially a neurostimulation system for a patient suffering from motoric dysfunction and/or autonomic dysfunction requires programming to define which stimulation settings need to be used to evoke certain muscles or muscle groups. Such muscles and/or muscle groups may be responsible for locomotion of the arms or legs, and/or responsible for e.g. bowel movement, sphincter control, bladder control and/or sexual function. Such programming of stimulation parameters may be performed by a user, such as a clinical professional, a physiotherapist and/or the patient himself, and facilitated by a computer-driven application. Neurostimulation, in particular multi-channel and/or variable neurostimulation often requires an interface to create the stimulation program and a stimulation system to deliver the stimulation. A user selects specific electrodes via a user interface on which he would like to perform stimulation for a patient who's implanted with the lead comprising the electrode array.

EP3527258A1 discloses a system for an electrical neurostimulator coupled to a plurality of electrodes, comprising: a user-controlled input device configured for generating directional control signals; and control circuitry configured for sequentially defining a plurality of different ideal bipole/tripole configurations relative to the plurality of electrodes In response to the directional control signals, generating a plurality of stimulation parameter sets respectively corresponding to the plurality of ideal bipole/tripole configurations, each stimulation parameter set defining relative amplitude values for the plurality of electrodes that emulate the respective ideal bipole/tripole configuration, and Instructing the electrical neurostimulator to convey electrical energy to the plurality of electrodes in accordance with the plurality of stimulation parameter sets.

EP3285855B1 discloses a system for delivering neurostimulation including a programming control circuit and a user interface. The programming control circuit may be configured to generate stimulation parameters controlling delivery of neurostimulation pulses according to one or more stimulation waveforms associated with areas of stimulation each defined by a set of electrodes. The neurostimulation pulses are each delivered to an area of stimulation.

The user interface may include a display screen and an Interface control circuit. The interface control circuit may be configured to define the one or more stimulation waveforms and the areas of stimulation and may include a stimulation frequency module configured to display a stimulation rate table on the display screen. The stimulation rate table may present stimulation frequencies associated with each of the areas of stimulation for selection by a user.

Currently, leads comprising an electrode array comprising multiple electrodes with an intended use for neuromodulation offer a limited range of freedom since their resolution is confined by the number of electrodes. Options such as unipolar and multipolar stimulation increase field-steering capabilities, but the user Is still limited by the fixed set of electrodes that he or she can pick from.

It is therefore an object of the present invention to provide a neuromodulation system which allows increasing neuromodulation options on a lead comprising an electrode array comprising electrodes.

This object is solved according to the present invention by a planning and/or control system for a system for providing neuromodulation, especially neurostimulation, at least comprising:
- a graphical presentation module configured and arranged for providing graphical information about an electrode array comprising multiple electrodes and/or an Implantation side for the electrode array comprising at least one target area,
- a selection module configured and arranged for determining a stimulation zone and/or a stimulation direction on the electrode array comprising at least one electrode and/or for individually selecting at least one electrode and/or for selecting at least one target area,
- a calculation module configured and arranged for determining a contribution of currents provided by electrodes of the stimulation zone and/or stimulation direction on the electrode array and/or the at least one electrode selected and/or to the at least one target area selected.

The invention is based on the basic Idea that the amount of stimulation options and/or stimulation resolution of a lead comprising an electrode array may be increased by using the concept of electrode and/or current weighting. Thus, the options for choosing stimulation options may no longer be constricted by selecting discrete electrodes (that means separate electrodes or one or more single electrode(s)) on a lead and/or electrode array but leverages the wide freedom of current weighting/balancing and presenting this in an Intuitive way to a user using the system. Alternatively, and/or additionally, combining stimulation options with imaging may allow a user to create a root for stimulation or steering current to more naturally stimulate nerves rather than creating homogenous fields. The use of a general concept including a graphical presentation module, a selection module and a calculation module combined into one strategy and made available for a system for providing neuromodulation, especially neurostlmulation may enable to reduce limitations of stimulation options with current leads and/or electrode arrays and enable that the patient may receive best possible stimulation.

In particular, the system may use anatomical information about the dorsal root trajectory, to adapt the weighing of electrode currents, to steer the field, such (parallel to the trajectories) that maximum activation Is obtained at target roots, while avoiding activation of the non-targeted roots.

The graphical presentation module may provide graphical information about an electrode array comprising actual physical electrodes and/or virtual electrodes. In particular, actual physical electrodes may reflect actual physical electrodes of a corresponding neuromodulation system related to the system for planning and/or control system. In particular, virtual electrodes may be represented by the graphical presentation module of the system only and may not reflect actual physical electrodes of a corresponding neuromodulation system related to the panning and/or control system according to the present invention. In other words, virtual electrodes may be Introduced by the system without physically introducing more. In particular, any number of virtual electrodes may be introduced in addition to the actual physical electrodes. In particular, virtual electrodes presented to a user may be more understandable and graspable than current-weighting or current-steering in percentages or absolute numbers. Overall, this enhances the options for choosing stimulation options.

In particular, each virtual and/or physical electrode may be controlled Independently. In particular, this may enable that each virtual and/or physical electrode may be characterized by a different frequency and/or waveform.

In particular, the user may be e.g. a therapist, a physiotherapist, a physician, a trainer, a medical professional, a patient and/or any person related to the patient.

In particular, the target area may be or may comprise fictlous and/or realistic anatomical conditions of a mammal, in particular a human being, such as the patient himself or a healthy individual. In particular, the target area may be or may comprise at least one target nerve and/or nerve fiber and/or dorsal root and/or area of the spinal cord and/or tissue and/or area related to the spinal cord and/or muscle fiber and/or muscle.

In particular, the target area could be augmented with an approximate neuronal model, e.g. neuronal fibers passing through dorsal roots. In particular, the system may enable to calculate contribution of currents based on neuronal simulations and/or anatomical conditions.

The selection module may be or may comprise a user Interface. In particular, the user interface may be a graphical user Interface. In particular, the selection module may be an input module. The selection module may comprise a mouse and/or a trackball and/or a joystick, a display and/or a touch screen and/or a touch pad and/or an acoustic signal and/or acoustic tone and/or a verbal command input. In particular, the selection module may be configured and arranged for allowing a user to actuate at least one control element, including but not limited to axes, points, knots, buttons, arrows, hand signals, emojis, crosses and/or windows and/or text and/or shortcuts in order to modify graphical information.

In particular, the selection module may enable to select Information and/or links and/or strategies provided by the graphical presentation module.

In particular, the stimulation zone may be or may comprise an area on a lead and/or electrode array comprising multiple electrodes, wherein the area comprises at least one electrode.

In particular, a stimulation zone may also be or may comprise a stimulation vector and/or a stimulation direction and/or at least one single electrode.

The calculation module may be or may comprise at least one algorithm. In particular, the calculation module may translate a selected stimulation zone and/or stimulation direction and/or the one or more electrodes individually selected and/or the at least one target area selected into a certain weighting, such as current weighting, on a real electrode array comprising electrodes, for stimulating a patient.

The calculation module may be configured and arranged for determining an equal contribution of currents provided by the electrodes of the stimulation zone and/or stimulation direction and/or the one or more electrodes individually selected and/or to the at least one target area selected. In particular, a user may select a stimulation zone and/or a stimulation direction and/or one or more electrodes individually and current sourcing may be distributed equally over all electrodes and/or anodes selected. Alternatively, and/or additionally, a user may select at least one target area and current sourcing may be distributed equally over the electrodes located at the at least one target area. In particular, this has the advantage that differences in excitation and/or excitability of different target nerves during equal stimulation may identified, which finally may enable optimizing stimulation protocols.

The calculation module may be configured and arranged for determining a weighted contribution of currents provided by the electrodes of the stimulation zone and/or stimulation direction and/or the at least one electrode individually selected and/or to the at least one target area selected. Overall, weighted contribution of currents, compared to homogenous fields, may enable more effective and closer to natural stimulation. In particular, weighted contribution may enable to direct a current to overcome neurological activity thresholds in an optimized matter. In particular, the second spatial derivative, tangential to a neuron/nerve may be the driving force behind activation. A simple field normal to a fiber may not lead to predictive activation, yet a tangent directional field may do.

In particular, for a stimulation direction, such as a stimulation vector, the weighting of electrodes may comprise distance and/or direction, in particular with the origin of the stimulation direction as the reference.

The calculation module may be configured and arranged for determining the weighted contribution of currents provided by the electrodes of the stimulation zone and/or stimulation direction by calculating the Euclidean distance from an electrode to the stimulation zone and/or stimulation direction and/or to at least one point of the stimulation zone and/or stimulation direction.

In particular, the Euclidean distance may be the ordinary distance between two points, here two electrodes and/or points of two electrodes. In particular, it may be the straight-line distance between two points and/or two electrodes and/or two points of electrodes. In particular, in a stimulation zone comprising more than two electrodes, this may have the advantage that the distance and the weighting of two electrodes is not affected by the addition of a third electrode to the analysis. Overall, weighting of each electrode of a stimulation zone and/or stimulation direction, based on the absolute and/or relative distance of the respective electrode of the stimulation zone and/or stimulation direction to one point of the stimulation zone and/or stimulation direction may be enabled. This may enable that e.g. decreased stimulation intensity is provided, the farer an electrode of the stimulation zone is from a specific stimulation center of a stimulation zone. This may enable close to natural stimulation.

In particular, the Euclidean distance may be the ordinary distance between two points, here e.g. two nerves and/or nerve fibers and/or parts of nerves and/or parts of nerve fibers of at least one target area. In particular, it is the straight-line distance between e.g. two nerves and/or nerve fibers and/or parts of nerves and/or parts of nerve fibers of at least one target area. Overall, weighting of nerves and/or nerve fibers and/or parts of nerves and/or parts of nerve fibers of at least one target area, based on the absolute and/or relative distance of the respective nerve and/or nerve fiber and or part of a nerve and/or part of a nerve fiber of the of the target area to one point of the target area may be enabled. This may enable that e.g. decreased stimulation intensity is provided, the farer a nerve and/or nerve fiber and/or part of nerve and/or part of nerve fiber of at least one target area Is from a specific stimulation center of a target area. This may enable close to natural stimulation.

The calculation module may be configured and arranged for determining the weighted contribution of currents provided by the electrodes of the stimulation zone and/or the stimulation direction and/or at least one electrode individually selected based on a generated field of neighbor electrodes. In particular, this may enable soft transitions of the current strengths of adjacent electrodes. Overall, this may enable close to natural stimulation.

The calculation module may be configured and arranged for determining the weighted contribution of currents provided by the electrodes of the stimulation zone and/or the stimulation direction and/or the at least one electrode individually selected and/or to the at least one target area selected by a numerical method. In particular, this approach may enable using a personalized patient model comprising the targeted and unwanted nerve locations (crosstalk), and which has computed for N electrode configurations the 3D potential distributions, as well as the activation potentials and/or selectivity indices. In particular, based on the stimulation zone and/or the stimulation direction and/or the at least one electrode individually selected and/or the at least one target area selected the numerical method may optimize the weighting of electrode intensities for maximizing a selectivity index, or for maximizing the target while minimizing sensitive areas. Overall, this may enable stimulation adapted to patient specific needs, i.e. patient specific stimulation.

In principle, it may be additionally and/or alternatively possible that different frequencies and/or pulse with are applied to different electrodes to create a stimulation zone. In particular, a user may select a certain zone and intends it to have 130 Hz stimulation, which may then be achieved by delivering differing stimulation frequencies to its surrounding electrodes, and the frequency of 130Hz in the stimulation zone may be achieved by amplification (overlapping, or constructive Interference) or cancellation Interference.

Further, the system may decide for which intended muscles/roots, a certain waveform should be used, e.g. a low frequency, a higher frequency, or a burst (e.g. triplets/quadruplets, with different burst frequency) waveform. In this case, the system may use knowledge of neurophysiology, i.e. which muscles (agonists/antagonists) and/or roots to determine if a higher frequency, or a burst frequency is required to Increase activation of target muscles and/or roots, while keeping unwanted muscles and/or roots less exposed.

The calculation module may be configured and arranged to feature an algorithm to determine the weighted contribution of currents to the benefit of power efficiency. This algorithm may be appended to any of the previously mentioned calculation module implementations. This algorithm may Interpret the input weighted electrode configuration and optimize it such that a new configuration is created that creates a considerably similar electrical field with the same effect on the effectuated neural tissue, yet with better power efficiency. In particular, with the increased stimulation freedom offered to the system by the 'Virtual Electrodes' concept, the search-space for more power-efficient settings to the algorithm is also increased. In addition to the algorithm altering the current weighting, the algorithm may further achieve power-efficiency effects by combining this with reduction or Increase of the amount of allocated electrodes (to reduce impedance), or may update the required stimulation current or total charge required to achieve the neurostimulation.

The system may further comprise at least one computer-assisted module configured and arranged for at least partially automatically determining a stimulation zone and/or a stimulation direction on the electrode array comprising at least one electrode and/or for at least partially automatically selecting at least one electrode, preferably based on medical imaging superimposing. In particular, medical Images may be obtained by MRI, CT, X-Ray, photography, etc. Alternatively, and/or additionally, anatomical models could be used. In particular, this may enable finding optimal zones and/or targets for stimulation, specifically adapted to the patient's anatomy and needs.

In other words, in general, the stimulation direction and/or stimulation zone on an electrode array comprising at least one electrode may be either selected manually by a user or automatically or semi-automatically by identifying a target area from anatomical data.

So, especially the following use-case is possible:On the visualization of the electrode overlapped on the anatomical model (with roots visible), we should be able to draw the stimulation zone (and direction) desired in order to target the roots of interest.
Furthermore this zone could be determined automatically If a software identifies the roots automatically from the anatomical model (e.g. MRI).

The system may further comprise at least one of a display, a controller, a programmer, a communication module, a telemetry module, a stimulation device, an electrode, a sensor and/or a sensor network.

In particular, the system may be a closed-loop system or an open-loop system.

It is also possible that the system allows both closed-loop or open loop functionality. In this regard, the user may switch between these options or there may be routines or control elements that can do or propose such a switch from closed-loop to open-loop and vice versa.

In particular, the system may be related to a system for providing neuromodulation, in particular neurostimulation to a patient. The system for providing neuromodulation, in particular neurostimulation to a patient may comprise at least one of a controller, a programmer, a communication module, a telemetry module, a stimulation device comprising an electrode array comprising multiple electrodes, a sensor and/or a sensor network.

According to the present invention a method is disclosed, the method characterized in that the method is performed with the system of any of claims 1-10.

In particular, a method for planning neuromodulation, especially neurostimulation, at least comprising the steps of:
- providing graphical information about an electrode array comprising multiple electrodes and/or an implantation side for the electrode array comprising at least one target area,
- determining a stimulation zone and/or a stimulation direction on the electrode array comprising at least one electrode and/or Individually selecting at least one electrode and/or selecting at least one target area,
- determining a contribution of currents provided by electrodes of the stimulation zone on the electrode array and/or the at least one electrode selected and/or to the at least one selected target area.

In particular, the graphical information about the electrode array may comprise actual physical electrodes and/or virtual electrodes.

The method may further comprise the step of determining an equal contribution of currents provided by the electrodes of the stimulation zone and/or the stimulation direction and/or at least one electrode individually selected and/or to the at least one target area selected.

In particular, the method may further comprise the step of determining a weighted contribution of currents provided by the electrodes of the stimulation zone and/or the stimulation direction and/or at least one electrode individually selected and/or to the at least one target area selected.

The method may further comprise the step of determining the weighted contribution of currents provided by the electrodes of the stimulation zone and/or the stimulation direction by calculating the Euclidean distance from an electrode to the stimulation zone and/or the stimulation direction and/or to at least one point of the stimulation zone and/or stimulation direction.

The method may further comprise the step of determining the weighted contribution of currents provided by the electrodes of the stimulation zone and/or the stimulation direction and/or the at least one electrode individually selected based on a generated field of neighbor electrodes.

The method may further comprise the step of determining the weighted contribution of currents provided by the electrodes of the stimulation zone and/or the stimulation direction and/or at least one electrode Individually selected and/or to the at least one target area selected by a numerical method.

The method may further comprise the step of least partially automatically determining a stimulation zone and/or a stimulation direction on the electrode array comprising at least one electrode and/or for at least partially automatically selecting at least one electrode, preferably based on medical imaging superimposing.

The method may further comprise the step of featuring an algorithm to determine the weighted contribution of currents to the benefit of power efficiency.

Further details and advantages of the present invention shall now be disclosed in connection with the drawings.

It is shown in:
- Fig. 1: a schematical overview of an embodiment of the planning and/or control system for a system for providing neuromodulation, especially neurostimulation according to the present invention, with which the method according to the present invention may be performed;
- Fig. 2: an example of an equal contribution of currents provided by individually selected electrodes of an electrode array, according to the present invention;
- Fig. 3: an example of weighted contribution of currents provided by electrodes of a determined stimulation zone of an electrode array, according to the present invention;
- Fig. 4: an example of an embodiment of an electrode array, comprising virtual electrodes, according to the present invention;
- Fig. 5: an example of determining a stimulation direction, according to the present invention;
- Fig. 6: two examples of graphical information provided by the graphical presentation module, combining an electrode array with a target area, according to the present Invention;
- Fig. 7: a further example of graphical information provided by the graphical presentation module, combining an electrode array with at least one possible target area, according to the present invention; and
- Fig. 8: a further example of graphical information provided by the graphical presentation module, according to the present invention.

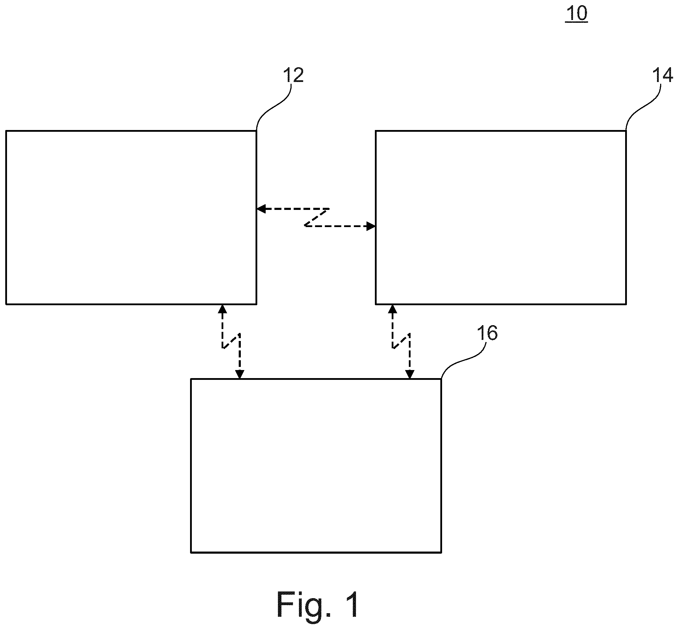

Fig. 1 shows a schematical overview of an embodiment of the planning and/or control system 10 for a system for providing neuromodulation, especially neurostimulation according to the present invention, with which the method according to the present invention may be performed.

The system 10 comprises a graphical presentation module 12.

In this embodiment, the graphical presentation module 12 is configured and arranged for providing graphical information about an electrode array A comprising multiple electrodes E, V and/or an Implantation side for the electrode array A comprising at least one target area X.

The system 10 further comprises a selection module 14.

In this embodiment, the selection module 14 is configured and arranged for determining a stimulation zone Z and/or a stimulation direction D on the electrode array A comprising at least one electrode E, V and/or for individually selecting at least one electrode E, V and/or for selecting at least one target area X.

The system 10 further comprises a calculation module 16.

In this embodiment, the calculation module 16 Is configured and arranged for determining a contribution of currents provided by electrodes E, V of the stimulation zone Z and/or stimulation direction D on the electrode array A and/or the at least one electrode E, V selected and/or a to the at least one target area X selected.

In this embodiment, the graphical presentation module 12, the selection module 14 and the calculation module 16 are connected.

In this embodiment, the graphical presentation module 12, the selection module 14 and the calculation module 16 are connected via a bidirectional connection.

In this embodiment, the graphical presentation module 12, the selection module 14 and the calculation module 16 are connected via a wireless link.

However, in an alternative embodiment, a unidirectional and/or cable-bound connection between the graphical presentation module 12, the selection module 14 and/or the calculation module 16 could be generally possible.

In this embodiment, the graphical presentation module 12 provides graphical Information about an electrode array A comprising multiple electrodes E, V.

In an alternative embodiment, the graphical presentation module 12 could provide additionally and/or alternatively graphical information abound an implantation side for the electrode array A comprising at least one target area X.

Not shown in this embodiment is that the graphical information about the implantation site could be patient-specific data.

Not shown in this embodiment is that the graphical information about the implantation site could be patient-specific MRI data, X-Ray data, pictures obtained during surgery, etc.

In this embodiment, the selection module 14 determines a stimulation zone Z.

In this embodiment, the selection module 14 determines a stimulation zone Z based on a user input.

Not shown in this embodiment is that the selection module 14 could generally comprise a user interface.

In an alternative embodiment, the selection module 14 could additionally and/or alternatively determine a stimulation direction D on the electrode array A comprising at least one electrode E, V and/or for selecting at least one target area X, based on user input.

Not shown in Fig. 1 is that the system 10 could further comprise at least one of a display, a controller, a programmer, a communication module, a telemetry module, a stimulation device, an electrode, a sensor and/or a sensor network.

Not shown in this embodiment is that the graphical presentation module 12 could in general provide graphical information about an electrode array A comprising actual physical electrodes E and/or virtual electrodes V.

Not shown in this embodiment Is that virtual electrodes V could be selected for stimulation.

Not shown In Fig. 1 is that virtual electrodes V may be more understandable and graspable to the user than current-weighting or current-steering in percentages and/or absolute numbers.

Not shown in Fig. 1 Is that it could be generally possible that the calculation module 16 could be configured and arranged for determining an equal contribution of currents provided by the electrodes E, V of the stimulation zone Z and/or the stimulation direction D and/or the one or more electrodes E, V individually selected and/or to the at least one target area X selected.

Not shown in Fig. 1 is that it could be generally possible that the calculation module 16 could be configured and arranged for determining a weighted contribution of currents provided by the electrodes E, V of the stimulation zone Z and/or stimulation direction D and/or the at least one electrode E, V individually selected and/or to the at least one target area X selected.

Not shown in Fig. 1 is that it could be generally possible that the calculation module 16 could be configured and arranged for determining the weighted contribution of currents provided by the electrodes E, V of the stimulation zone Z and/or stimulation direction D by calculating the Euclidean distance from an electrode E, V to the stimulation zone Z and/or stimulation direction D and/or to at least one point of the stimulation zone Z and/or stimulation direction D.

Further not shown in Fig. 1 is that It could be generally possible that the calculation module 16 could be configured and arranged for determining the weighted contribution of currents provided by the electrodes E, V of the stimulation zone Z and/or the stimulation direction D and/or at least one electrode E, V individually selected based on a generated field of neighbor electrodes E, V.

In particular, from an activation line such as a stimulation vector and/or a stimulation direction D, it could be calculated which neighboring electrodes E, V can create a field In this direction and weighting factors could be determined.

Further not shown In Fig. 1 Is that It could be generally possible that the calculation module 16 could be configured and arranged for determining the weighted contribution of currents provided by the electrodes E, V of the stimulation zone Z and/or the stimulation direction D and/or the at least one electrode E, V individually selected and/or to the at least one target area X selected by a numerical method.

In general, instead of analytical models, a numerical method could be used, using a personalized patient model M, e.g. a MRI based patient model M, that includes both targeted and unwanted (cross-talk) nerve locations, and which has computed for N electrode E, V configurations the 3D potential distributions, as well as the activation potentials/ selectivity Indices.

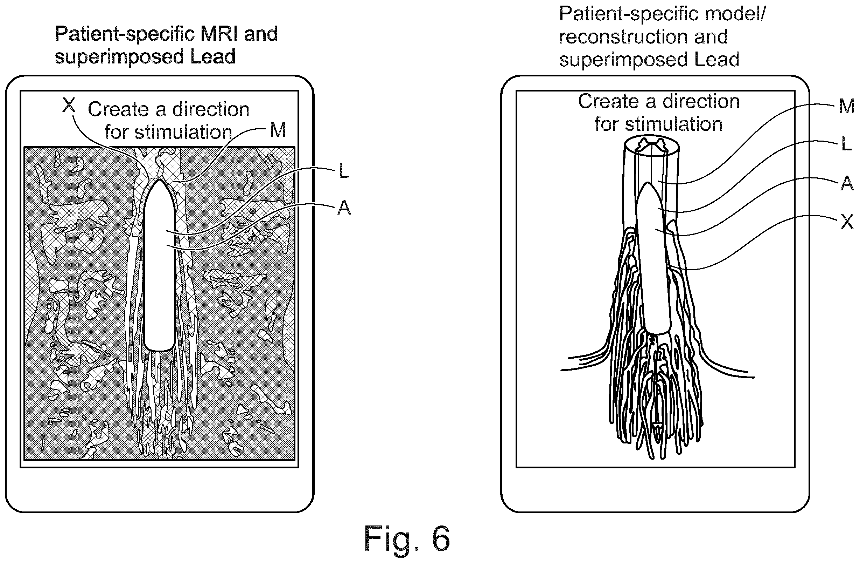

Not shown in Fig. 1 is that the system 10 could further comprise at least one computer-assisted module configured and arranged for at least partially automatically determining a stimulation zone Z and/or a stimulation direction D on the electrode array A comprising at least one electrode E, V and/or for at least partially automatically selecting at least one electrode E, V, preferably based on medical imaging superimposing, cf. Fig. 6.

Not shown in Fig. 1 is that the calculation module 16 could generally feature an algorithm to determine the weighted contribution of currents to the benefit of power efficiency.

Based on the electrodes E, V of the stimulation zone Z and/or the stimulation direction D and/or the at least one electrode E, V Individually selected and/or the at least one target area X indicated by the user, the calculation module 16 could use a (pre-computed, or online calculated) neuronal activation model, to optimize the weighting of electrode E, V Intensities for maximizing the selectivity index, or for maximizing the target while minimizing the sensitive areas.

In general, the system 10 could perform a method for planning neuromodulation, especially neurostimulation, at least comprising the steps of:
- providing graphical Information about an electrode array A comprising multiple electrodes E, V and/or an implantation side for the electrode array A comprising at least one target area X,
- determining a stimulation zone Z and/or a stimulation direction D on the electrode array A comprising at least one electrode E, V and/or individually selecting at least one electrode E, V and/or selecting at least one target area X,
- determining a contribution of currents provided by electrodes E, V of the stimulation zone Z and/or the stimulation direction D on the electrode array A and/or the at least one electrode E, V selected and/or to the at least one selected target area X.

In general, the graphical information about the electrode array A could comprise actual physical electrodes E and/or virtual electrodes V.

The method could further comprise the step of determining an equal contribution of currents provided by the electrodes E, V of the stimulation zone Z and/or the stimulation direction D and/or at least one electrode E, V individually selected and/or to the at least one target area X selected.

The method could further comprise the step of determining a weighted contribution of currents provided by the electrodes E, V of the stimulation zone Z and/or the stimulation direction D and/or at least one electrode E, V Individually selected and/or to the at least one target area X selected.

The method could further comprise the step of determining the weighted contribution of currents provided by the electrodes E, V of the stimulation zone Z and/or the stimulation direction D by calculating the Euclidean distance from an electrode E, V to the stimulation zone Z and/or the stimulation direction D and/or to at least one point of the stimulation zone Z and/or stimulation direction D.

The method cold further comprises the step of determining the weighted contribution of currents provided by the electrodes E, V of the stimulation zone Z and/or the stimulation direction D and/or the at least one electrode E, V individually selected based on a generated field of neighbor electrodes E, V.

The method could further comprise the step of determining the weighted contribution of currents provided by the electrodes E, V of the stimulation zone Z and/or the stimulation direction D and/or at least one electrode E, V Individually selected and/or to the at least one target area X selected by a numerical method.

The method could further comprise the step of least partially automatically determining a stimulation zone Z and/or a stimulation direction D on the electrode array A comprising at least one electrode E, V and/or for at least partially automatically selecting at least one electrode E, V, preferably based on medical imaging superimposing.

The method could further comprise the step of featuring an algorithm to determine the weighted contribution of currents to the benefit of power efficiency.

In general, the weighting may be presented as absolute numbers (e.g. in V, mA, A) and/or expressed as percentage of total current applied by involved electrodes E, V.

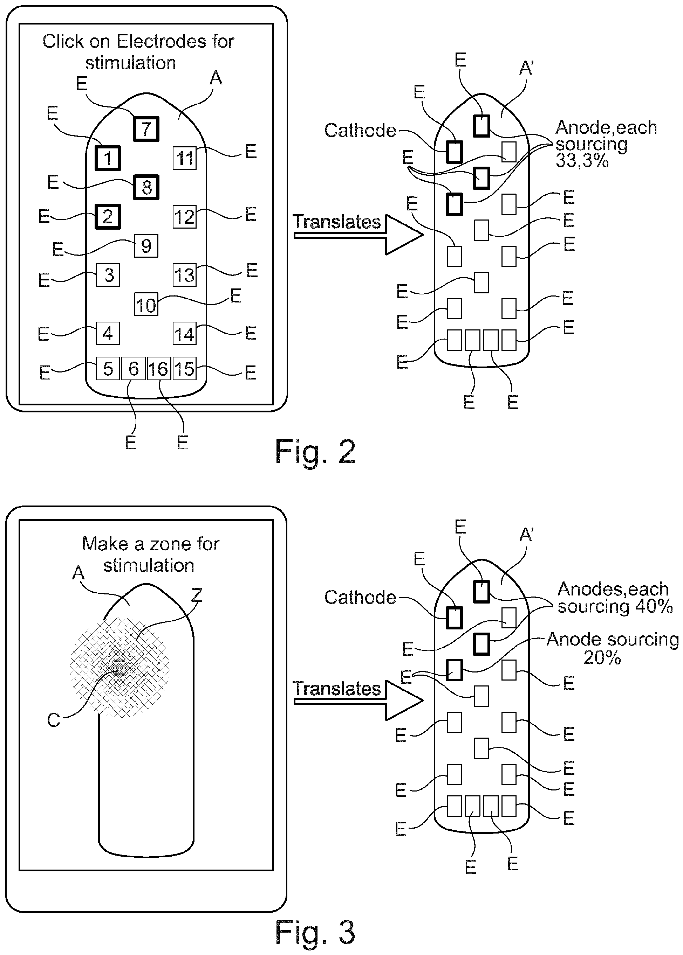

Examples of how to determine a stimulation zone Z and/or stimulation direction D and/or select individual electrodes E, V and/or at least one target area X are disclosed In Figs 2, 3, 4, 5, 6, 7 and/or 8.

Fig. 2 shows an example of an equal contribution of currents provided by individually selected electrodes E, V of an electrode array A, determined by the calculation module 16 of the system 10 disclosed in Fig. 1.

In this embodiment, the graphical Information provided by the graphical presentation module 12 of the system 10 disclosed in Fig. 1 comprises an electrode array A comprising 16 electrodes E.

In this embodiment, the system 10 Is connected to a system for neuromodulation, comprising an electrode array A'.

In this embodiment, the electrode array A comprising 16 electrodes E provided by the graphical presentation module 12 reflects an electrode array A' of a related neuromodulation system configured and arranged for providing neuromodulation to a patient.

In this embodiment, the selection module 14 comprises a user interface.

In this embodiment, the selection module 14 comprises a graphical user interface.

In this embodiment, the selection module 14 is configured and arranged for individually selecting at least one electrode E of the electrode array A.

In this embodiment, the selection module 14 Is configured and arranged for individually selecting at least one electrode E of the electrode array A based on a user input.

The shown embodiment Is a display of a tablet computer, in particular a touch screen.

However, in general, any display and/or touch screen and/or programmer and/or mobile device could be possible.

In this embodiment, the display discloses the graphical information provided by the graphical presentation module 12.

In this embodiment, the user selects electrodes E of the electrode array A' by touching with a finger on the touch screen, in particular on respective electrodes E of the electrode array A on the display.

Alternatively, the electrodes E could be selected via a mouse click.

In general, it could be possible that the electrodes E are selected by a mouse and/or a trackball and/or a joystick, a display and/or a touch screen and/or a touch pad and/or an acoustic signal and/or acoustic tone and/or a verbal command input.

In general, the selection module 14 could allow a user to actuate at least one control element, including but not limited to axes, points, knots, buttons, arrows, hand signals, emojis, crosses and/or windows and/or text and/or shortcuts.

In this embodiment, the user Input is translated by the calculation module 16 into a certain weighting that could then be sent to the neuromodulation system for providing neuromodulation.

In this embodiment, the neuromodulation provided by the system for providing neuromodulation Is modified according to the user input.

In this embodiment, the calculation module 16 of the system 10 disclosed In Fig. 1 determines an equal contribution of currents provided by three electrodes E individually selected.

In this embodiment, the calculation module 16 of the system 10 disclosed in Fig. 1 determines an equal contribution of currents provided by electrodes E 2, 7 and 8 individually selected.

In this embodiment, the calculation module 16 of the system 10 disclosed in Fig. 1 determines an equal contribution of currents provided by three anodes Individually selected.

In this embodiment, each selected anode comprises 33.33% current sourcing.

In this embodiment, the calculation module 16 translates electrodes E of an electrode array A individually selected into an equal weighting on a real electrode array A' comprising electrodes E, for stimulating a patient.

Not shown In Fig. 2 is that the electrode array A' of the related neuromodulation system could provide neuromodulation, in particular neurostimulation to a patient based on the determined current sourcing.

**Fig. 3** shows an example of weighted contribution of currents provided by electrodes E of a determined stimulation zone Z of an electrode array A, determined by the calculation module 16 of the system 10 disclosed in Fig. 1.

In this embodiment, the graphical information provided by the graphical presentation module 12 of the system 10 disclosed in Fig. 1 comprises an electrode array A.

In this embodiment, the electrode array A comprises 16 electrodes E.

In this embodiment, the electrode array A comprising 16 electrodes E provided by the graphical presentation module 12 reflects an electrode array A' of a neuromodulation system configured and arranged for providing neuromodulation to a patient.

In this embodiment, the selection module 14 comprises a user Interface.

In this embodiment, the selection module 14 comprises a graphical user interface.

In this embodiment, the selection module 14 is configured and arranged for determining at least one stimulation zone Z.

In this embodiment, the selection module 14 is configured and arranged for determining at least one stimulation zone Z based on a user input.

The shown embodiment is a display of a tablet computer, in particular a touch screen.

However, In general, any display and/or touch screen and/or programmer and/or mobile device could be possible.

In this embodiment, the display discloses the graphical information provided by the graphical presentation module 12.

In this embodiment, the user determines a stimulation zone Z by touching with a finger on the touch screen.

Alternatively, the stimulation zone could be determined via a mouse click.

In general, it could be possible that the stimulation zone and/or stimulation direction D is determined by a mouse and/or a trackball and/or a joystick, a display and/or a touch screen and/or a touch pad and/or an acoustic signal and/or acoustic tone and/or a verbal command input.

In general, the selection module 14 could allow a user to actuate at least one control element, including but not limited to axes, points, knots, buttons, arrows, hand signals, emojis, crosses and/or windows and/or text and/or shortcuts.

This user Input could generally be translated by the calculation module 16 into a certain weighting that could then be sent to a neuromodulation system for providing neuromodulation.

In this embodiment, the system 10 is connected to a system for neuromodulation, comprising an electrode array A'.

In this embodiment, the user input is translated by the calculation module 16 into a certain weighting that could then be sent to the neuromodulation system for providing neuromodulation.

In this embodiment, the neuromodulation provided by the system for providing neuromodulation can be modified according to a modification of the user input.

In this embodiment, the calculation module 16 of the system 10 disclosed in Fig. 1 determines
a weighted contribution of currents provided by the electrodes E of the stimulation zone Z.

In this embodiment, the calculation module 16 of the system 10 disclosed In Fig. 1 determines
a weighted contribution of currents provided by the electrodes E of the stimulation zone Z, with two anodes comprising 40% of current sourcing and one anode comprising 20% of current sourcing.

Not shown In this embodiment is that the weighted contribution of currents provided by the electrodes E of the stimulation zone Z is determined by calculating the Euclidean distance from the electrodes E to the center C of the stimulation zone Z.

Not shown in this embodiment is that the weighted contribution of currents provided by the electrodes E of the stimulation zone Z is determined by calculating the Euclidean distance from the electrodes E to any other point on the electrode array A, including the stimulation zone Z.

In this embodiment, the calculation module 16 translates the selected stimulation zone Z of an electrode array A into a different weighting on a real electrode array A' comprising electrodes E, for stimulating a patient.

Not shown in Fig. 3 is that the electrode array A' of the related neuromodulation system could provide neuromodulation, In particular neurostimulation to a patient based on the determined current sourcing.

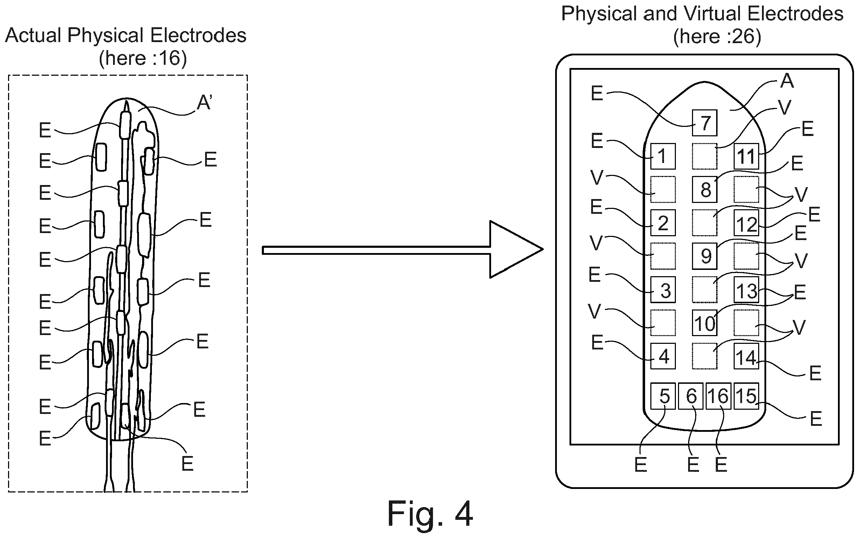

**Fig. 4** shows an example of an embodiment of an electrode array A, comprising virtual. electrodes V, according to the present invention.

In this embodiment, the system 10 is connected to a system for neuromodulation, comprising an electrode array A' (actual physical electrode array A' comprising actual physical electrodes E).

The shown example shows an actual physical electrode array A' (left) as well as a display of a tablet computer, in particular a touch screen (right).

In this embodiment, the display discloses the graphical information provided by the graphical presentation module 12.

However, in general, any display and/or touch screen and/or programmer and/or mobile device could be possible.

In this embodiment, the graphical information provided by the graphical presentation module 12 of the system 10 disclosed in Fig. 1 comprises an electrode array A.

In this embodiment, the graphical Information provided by the graphical presentation module 12 of the system 10 disclosed in Fig. 1 comprises an electrode array A, comprising multiple electrodes E, V.

In this embodiment, the electrode array A disclosed by the graphical presentation module 12 comprises 16 physical electrodes E.

In this embodiment, the electrode array A' of the actual physical electrode array A comprises 16 physical electrodes E.

In an alternative embodiment, the electrode array A could comprise any other number of physical electrodes E.

In this embodiment, the 16 physical electrodes E of the electrode array A relate to the 16 physical electrodes of the actual physical electrode array A' comprised in the neuromodulation system related to the system 10 disclosed in Fig. 1.

In this embodiment, the electrode array A comprises 10 virtual electrodes V.

In this embodiment, the 10 virtual electrodes V do not relate to physical electrodes of an actual physical electrode array A' comprised in a neuromodulation system related to the system 10 disclosed in Fig. 1.

In other words, the 10 virtual electrodes V allow to go from 16 electrodes E present on an electrode array A to 26 electrodes E, V.

Not shown in Fig. 4 is that any number of virtual electrodes V could be added to the electrodes E.

In this embodiment, the selection module 14 comprises a user interface.

In this embodiment, the selection module 14 comprises a graphical user interface.

In this embodiment, the selection module 14 is configured and arranged for selecting electrodes E, V.

In this embodiment, the selection module 14 is configured and arranged for selecting electrodes E, V based on a user input.

In this embodiment, the user determines a stimulation zone Z on the electrode array A' by selecting actual physical electrodes E and virtual electrodes V on the electrode array A shown on the display.

In this embodiment, the stimulation zone Z is determined by selecting electrodes E, V via a mouse click.

In general, it could be possible that the stimulation zone and/or a stimulation direction D is determined by a mouse and/or a trackball and/or a joystick, a display and/or a touch screen and/or a touch pad and/or an acoustic signal and/or acoustic tone and/or a verbal command input.

In general, the selection module 14 could allow a user to actuate at least one control element, including but not limited to axes, points, knots, buttons, arrows, hand signals, emojis, crosses and/or windows and/or text and/or shortcuts.

This user Input could generally be translated by the calculation module 16 into a certain weighting that could then be sent to a neuromodulation system for providing neuromodulation.

This user input, thus the selection of physical electrodes E and virtual electrodes V on an electrode array A could generally be translated by the calculation module 16 Into a certain weighting (equal or unequal) that could then be sent to a neuromodulation system for providing neuromodulation via an electrode array A' comprising physical electrodes E.

Not shown in Fig. 4 is that the virtual electrodes V that can be selected for stimulation could be more understandable and graspable to the user than current-weighting or current-steering in percentages.

Not shown in Fig. 4 is that while selecting and/or interacting with virtual electrodes V, at least one key performance Indicator, such as the selectivity index could be provided, In particular as graphical information provided by the graphical presentation module 12.

Not shown In Fig. 4 Is that while selecting and/or Interacting with virtual electrodes V, at least one key performance indicator, such as the selectivity index could be provided dynamically, in particular as graphical information provided by the graphical presentation module 12.

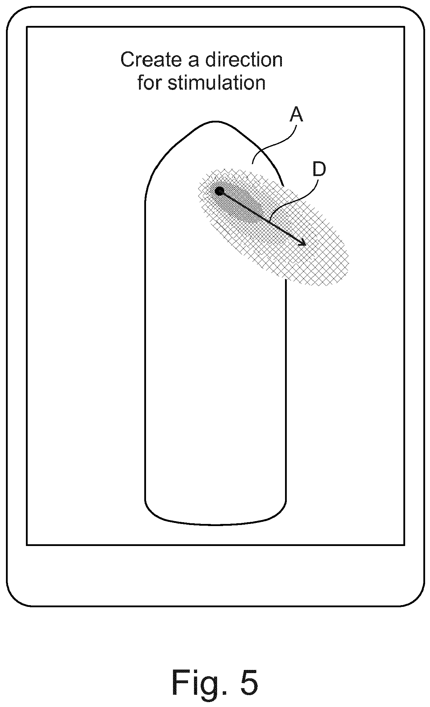

**Fig. 5** shows an example of determining a stimulation direction D, according to the present Invention.

The shown embodiment is a display of a tablet computer, in particular a touch screen.

However, in general, any display and/or touch screen and/or programmer and/or mobile device could be possible.

In this embodiment, the display discloses the graphical Information provided by the graphical presentation module 12.

In this embodiment, the graphical Information provided by the graphical presentation module 12 of the system 10 disclosed in Fig. 1 comprises an electrode array A.

In this embodiment, the selection module 14 comprises a user interface.

In this embodiment, the selection module 14 comprises a graphical user interface.

In this embodiment, the selection module 14 Is configured and arranged for determining a stimulation direction D.

In this embodiment, the selection module 14 Is configured and arranged for determining at least one stimulation direction D based on a user input.

In this embodiment, the user determines a stimulation direction D by touching with a finger on the touch screen and moving the finger.

In this embodiment, the stimulation direction D is a vector.

Alternatively, the stimulation direction D could be determined via a mouse click.

In general, it could be possible that the stimulation direction D Is determined by a mouse and/or a trackball and/or a joystick, a display and/or a touch screen and/or a touch pad and/or an acoustic signal and/or acoustic tone and/or a verbal command input.

In general, the selection module 14 could allow a user to actuate at least one control element, including but not limited to axes, points, knots, buttons, arrows, hand signals, emojis, crosses and/or windows and/or text and/or shortcuts.

This user input could generally be translated by the calculation module 16 Into a certain weighting that could then be sent to a neuromodulation system for providing neuromodulation.

Not shown in Fig. 5 is that the stimulation direction D on the electrode array A comprises electrodes E.

Not shown in Fig. 5 is that the calculation module 16 of the system 10 disclosed In Fig. 1 determines a weighted contribution of currents provided by the electrodes E of the stimulation direction D.

In general, the user input could generally be translated by the calculation module 16 into a certain weighting that could then be sent to a neuromodulation system for providing neuromodulatlon.

Not shown in Fig. 5 Is that the calculation module 16 of the system 10 disclosed in Fig. 1 could generally determine a weighted contribution of currents provided by the electrodes E of the stimulation direction D or an equal contribution of currents provided by the electrodes E of the stimulation direction D.

Fig. 6 shows two examples of graphical information provided by the graphical presentation module, combining an electrode array A with a target area X, according to the present invention.

The shown embodiment (both examples) Is a display of a tablet computer, in particular a touch screen.

However, in general, any touch screen and/or programmer and/or mobile device could be possible.

In this embodiment, the display discloses the graphical information provided by the graphical presentation module 12 of the system 10 disclosed in Fig. 1.

In this embodiment, the selection module 14 comprises a user interface.

In this embodiment, the selection module 14 comprises a graphical user Interface.

In this embodiment, the electrode array A is comprised in a lead L.

In this embodiment, the lead L is superimposed on a target area X.

In this embodiment, the graphical information provided by the graphical presentation module 12 of the system 10 disclosed in Fig. 1 comprises an electrode array A and a target area X.

Left: In this embodiment, the target area X Is provided as patient specific data obtained my MRI.

However, it could be generally possible, that the target area X is provided as patient specific data obtained by other methods, such as X-Ray, CT, or pictures obtained during surgery.

Right: In this embodiment, the target area X is a reconstruction.

In this embodiment, the target area X is a model M.

In general, the target area X could be provided as a 2D model M and/or a 3D model M.

Not shown in Fig. 6 Is that the user could place the electrode array A on at least one target area X by touching with his finger on the electrode array A and moving the electrode array A with his finger.

Not shown in Fig. 6 is that a computer-assisted module could at least partially automatically determining a stimulation zone Z and/or a stimulation direction D on the electrode array A comprising at least one electrode E, V and/or for at least partially automatically selecting at least one electrode E, V preferably based on the medical imaging superimposing and/or a model M.

It could be generally possible that the electrode array A Is superimposed on a medical image and/or model.

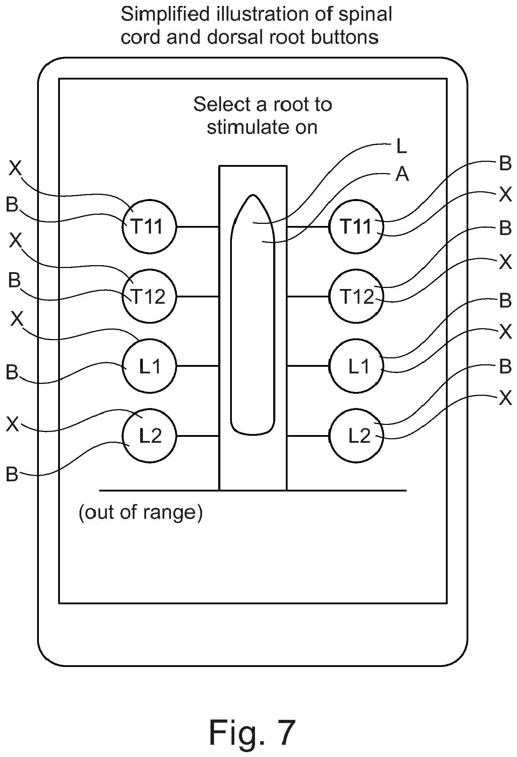

**Fig. 7** shows a further example of graphical information provided by the graphical presentation module, combining an electrode array A with at least one possible target area X, according to the present invention.

The shown embodiment (both examples) is a display of a tablet computer, In particular a touch screen.

However, in general, any touch screen and/or programmer and/or mobile device could be possible.

In this embodiment, the display discloses the graphical information provided by the graphical presentation module 12 of the system 10 disclosed In Fig. 1.

In this embodiment, the selection module 14 comprises a user Interface.

In this embodiment, the selection module 14 comprises a graphical user interface.

In this embodiment, the graphical Information provided by the graphical presentation module 12 of the system 10 disclosed in Fig. 1 comprises an electrode array A and different possible target areas X.

In this embodiment, the electrode array A Is comprised In a lead L.

In this embodiment, different possible target areas X with a superimposed lead L are shown.

In this embodiment, the different possible target areas X are shown as buttons B.

In this embodiment, a user can select at least one target area X by selecting at least one corresponding button B, e.g. by touching the corresponding button B with a finger.

Alternative ways of selection are disclosed for Fig. 1, 2, 3, 4, 5, 6.

In this embodiment, the different possible target areas X are provided schematically as spinal cord and pairs of spinal nerves, i.e. pairs of thoracic nerves T11, T12 and lumbar nerves L1, L2.

In an alternative embodiment, the different possible target areas X could alternatively and/or additionally Include muscles (e.g. leg/trunk muscles) and/or organs.

In general, muscles and/or organs could be positioned under an electrode array A and/or a lead L and/or or next to an electrode array A and/or a lead L.

However, in an alternative embodiment, the different possible target areas X could be provided as any spinal nerve and/or any combination of at least two spinal nerves.

In general, the at least one target area X could be or could comprise fictious and/or realistic anatomical conditions of a mammal, in particular a human being, such as the patient himself.

In general, the at least one target area X could be or could comprise at least one target nerve and/or nerve fiber and/or dorsal root and/or area of the spinal cord and/or tissue and/or area related to the spinal cord and/or muscle fiber and/or muscle.

In general, image data and/or 2D models M and/or 3D models M could be used as target area X for visual representation of anatomy of the patient, e.g. the dorsal roots.

In this embodiment, the user can select at least one target area X, i.e. left and/or right thoracic nerve T11 and/or T12 and/or left and/or right lumbar nerve L1 and/or L2.

In this embodiment, the user can select at least one target area X, i.e. left and/or right thoracic nerve T11 and/or T12 and/or left and/or right lumbar nerve L1 and/or L2 by selecting the respective button B.

In general, via a user interface comprised in the selection module 14, a target area X could be determined and weighted by the system 10.

Not shown in this embodiment is, that the calculation module 16 could translate the at least one target area X selected into a certain weighting, such as a current weighing, on a real electrode array A' comprising electrodes E related to the system 10 for stimulating a patient.

In general, the target area X could be alternatively and/or additionally provided as kinematic model M, wherein intended muscles could be determined and weighted and/or unwanted crosstalk between agonists and antagonists could be assigned.

Not shown in Fig. 7 is that while selecting and/or interacting with target areas, at least one key performance indicator, such as the selectivity Index could be provided, in particular as graphical information provided by the graphical presentation module 12.

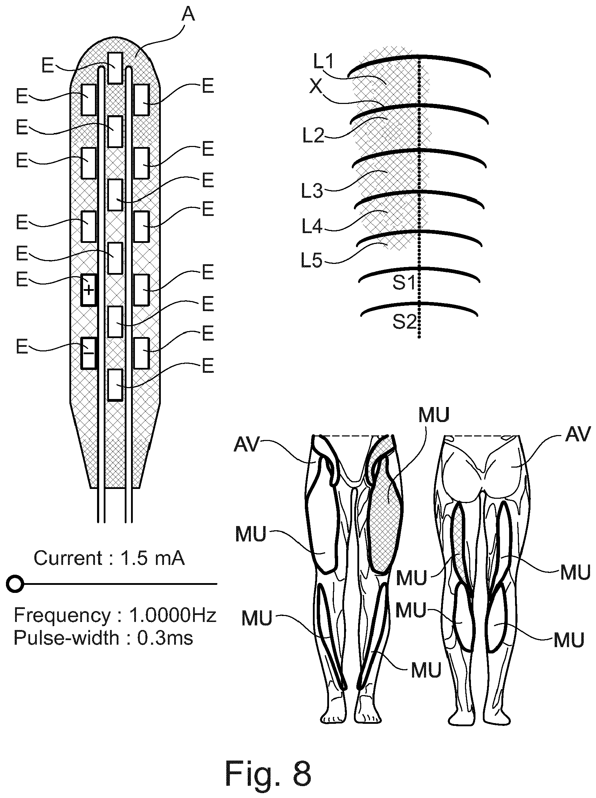

In general, it could also be possible that target muscles and/or planed movements are selected, and stimulation is applied according to the selection, cf. Fig. 8.

**Fig. 8** shows a further example of graphical information provided by the graphical presentation module 12, according to the present Invention.

The shown embodiment is a display of a tablet computer, in particular a touch screen.

However, in general, any touch screen and/or screen and/or mobile device could be possible.

In this embodiment, the display discloses the graphical information provided by the graphical presentation module 12 of the system 10 disclosed in Fig. 1.

In this embodiment, the selection module 14 comprises a user interface.

In this embodiment, the selection module 14 comprises a graphical user interface.

In this embodiment, the graphical Information provided by the graphical presentation module 12 of the system 10 disclosed In Fig. 1 comprises possible target muscles MU to be stimulated, illustrated as different muscles MU of an avatar AV.

In this embodiment, the graphical information provided by the graphical presentation module 12 of the system 10 disclosed in Fig. 1 further comprises a schema configured and arranged to illustrate a target area X based on target muscles MU.

in this embodiment, a user can select at least one target muscle MU by selecting at least one muscle MU, e.g. by touching the corresponding muscle MU of the avatar AV with a finger.

In this embodiment, the user selects the right quadriceps Q.

In this embodiment, the selection module 14, based on the selected target muscle MU, selects a target area X.

In this embodiment, the target area X comprises spinal nerves L1-L5.

Not shown in this embodiment is that a patient is implanted with an electrode array A (electrode array A also shown in Fig. 8) capable of providing stimulation on the target area X.

In this embodiment, the calculation module 16 determines a contribution of currents provided to the target area X selected in order to stimulate the muscle MU selected.

In this embodiment, the contribution of currents provided to the target area X gradually decreases from the center to the outside O of the target area X.

The electrode array A provides stimulation to the target area X via selected electrodes E and selected stimulation parameters.

In this example, the current is 1.5 mA, the frequency is 1.0000 Hz and the pulse-width Is 0.3 ms.

However, in an alternative embodiment, other stimulation parameters could be generally possible.

### References

- 10: system
- 12: graphical presentation module
- 14: selection module
- 16: calculation module

- A, A': electrode array
- AV: avatar
- B: button
- C: center
- D: stimulation direction
- E: (actual physical) electrode
- M: patient specific model
- MU: muscle
- L: lead
- L1: lumbar spinal nerve 1
- L2: lumbar spinal nerve 2
- L3: lumbar spinal nerve 3
- L4: lumbar spinal nerve 4
- L5: lumbar spinal nerve 5
- Q: quadriceps
- S1: sacral spinal nerve 1
- S2: sacral spinal nerve 2
- T11: thoracic spinal nerve 11
- T12: thoracic spinal nerve 12
- V: virtual electrode
- X: target area
- Z: stimulation zone

## Claims

1. A planning and/or control system (10) for a system for providing neuromodulation, especially neurostimulation, at least comprising:
- a graphical presentation module (12) configured and arranged for providing graphical information about an electrode array (A) comprising multiple electrodes (E, V) and/or an implantation side for the electrode array comprising at least one target area (X),
- a selection module (14) configured and arranged for determining a stimulation zone (Z) and/or a stimulation direction (D) on the electrode array (A) comprising at least one electrode (E, V) and/or for individually selecting at least one electrode (E, V) and/or for selecting at least one target area (X),
- a calculation module (16) configured and arranged for determining a contribution of currents provided by electrodes (E;V) of the stimulation zone (Z) and/or stimulation direction (D) on the electrode array (A) and/or the at least one electrode (E, V) selected and/or to the at least one target area (X) selected.

2. The system (10) according to claim 1, **characterized in that** the graphical presentation module (12) is configured and arranged for providing graphical information about an electrode array (A) comprising actual physical electrodes (E) and/or virtual electrodes (V).

3. The system (10) according to claim 1 or claim 2, **characterized in that** the calculation module (16) is configured and arranged for determining an equal contribution of currents provided by the electrodes (E, V) of the stimulation zone (Z) and/or stimulation direction (D) and/or the one or more electrodes (E, V) individually selected and/or to the at least one target area (X) selected.

4. The system (10) according to claim 1 or claim 2, **characterized in that** the calculation module (16) is configured and arranged for determining a weighted contribution of provided by the electrodes (E, V) of the stimulation zone (Z) and/or stimulation direction (D) and/or the one or more electrodes (E, V) individually selected and/or to the at least one target area (X) selected.

5. The system (10) according to claim 1, claim 2 or claim 4, **characterized in that** the calculation module (16) is configured and arranged for determining the weighted contribution of currents provided by the electrodes (E, V) of the stimulation zone (Z) and/or stimulation direction (D) by calculating the Euclidean distance from an electrode (E, V) to the stimulation zone (Z) and/or stimulation direction (D) and/or to at least one point of the stimulation zone (Z) and/or stimulation direction (D).

6. The system (10) according to claim 1, claim 2 or claim 4, **characterized in that** the calculation module (16) is configured and arranged for determining the weighted contribution of currents provided by the electrodes (E, V) of the stimulation zone (Z) and/or the stimulation direction (D) and/or at least one electrode (E, V) individually selected based on a generated field of neighbor electrodes (E, V).

7. The system (10) according to claim 1, claim 2 or claim 4, **characterized in that** the calculation module (16) is configured and arranged for determining the weighted contribution of currents provided by the electrodes (E, V) of the stimulation zone (Z) and/or the stimulation direction (D) and/or the at least one electrode (E, V) individually selected and/or to the at least one target area (X) selected by a numerical method.

8. The system (10) according to one of claims 1 to 7, **characterized in that** the system (10) further comprises at least one of a display, a controller, a programmer, a communication module, a telemetry module, a stimulation device, an electrode, a sensor and/or a sensor network.

9. The system (10) according to one of claims 1 to 8, **characterized in that** the system (10) further comprises at least one computer-assisted module configured and arranged for at least partially automatically determining a stimulation zone (Z) and/or a stimulation direction (D) on the electrode array (A) comprising at least one electrode (E, V) and/or for at least partially automatically selecting at least one electrode (E, V), preferably based on medical imaging superimposing.

10. The system (10) according to one of claims 1 to 9, **characterized in that** the calculation module (16) is be configured and arranged to feature an algorithm to determine the weighted contribution of currents to the benefit of power efficiency.

11. A method for planning neuromodulation, especially neurostimulation, at least comprising the steps of:
- providing graphical information about an electrode array (A) comprising multiple electrodes (E, V) and/or an implantation side for the electrode array (A) comprising at least one target area (X),
- determining a stimulation zone (Z) and/or a stimulation direction (D) on the electrode array (A) comprising at least one electrode (E, V) and/or individually selecting at least one electrode (E, V) and/or selecting at least one target area (X),
- determining a contribution of currents provided by electrodes (E, V) of the stimulation zone (Z) and/or the stimulation direction (D) on the electrode array (A) and/or the at least one electrode (E, V) selected and/or to the at least one selected target area (X).

12. The method according to claim 11, **characterized in that** the graphical information about the electrode array (A) comprises actual physical electrodes (E) and/or virtual electrodes (V).

13. The method according to claim 11 or claim 12, **characterized in that** the method further comprises the step of determining an equal contribution of currents provided by the electrodes (E, V) of the stimulation zone (Z) and/or the stimulation direction (D) and/or at least one electrode (E, V) individually selected and/or to the at least one target area (X) selected.

14. The method according to claim 11 or claim 12, **characterized in that** the method further comprises the step of determining a weighted contribution of currents provided by the electrodes (E, V) of the stimulation zone (Z) and/or the stimulation direction (D) and/or at least one electrode (E, V) individually selected and/or to the at least one target area (X) selected.

15. The method according to claim 11, claim 12 or claim 14, **characterized in that** the method further comprises the step of determining the weighted contribution of currents provided by the electrodes (E, V) of the stimulation zone (Z) and/or the stimulation direction (D) by calculating the Euclidean distance from an electrode (E, V) to the stimulation zone (Z) and/or the stimulation direction (D) and/or to at least one point of the stimulation zone (Z) and/or at least one stimulation direction (D).

16. The method according to claim 11, claim 12 or claim 14, **characterized in that** the method further comprises the step of determining the weighted contribution of currents provided by the electrodes (E, V) of the stimulation zone (Z) and/or the stimulation direction (D) and/or the at least one electrode (E, V) individually selected based on a generated field of neighbor electrodes (E, V).

17. The method according to claim 11, claim 12 or claim 14, **characterized in that** the method further comprises the step of determining the weighted contribution of currents provided by the electrodes (E, V) of the stimulation zone (Z) and/or the stimulation direction (D) and/or at least one electrode (E, V) individually selected and/or to the at least one target area (X) selected by a numerical method.

18. The method according to any of the preceding claims, **characterized in that** the method further comprises the step of least partially automatically determining a stimulation zone (Z) and/or a stimulation direction (D) on the electrode array (A) comprising at least one electrode (E, V) and/or for at least partially automatically selecting at least one electrode (E, V), preferably based on medical imaging superimposing.

19. The method according to any of the preceding claims, **characterized in that** the method further comprises the step of featuring an algorithm to determine the weighted contribution of currents to the benefit of power efficiency.
